# EUROPEAN PATENT APPLICATION

(11) **EP 1 629 837 A1**
(43) Date of publication of application: **01.03.2006**
(21) Application number: 04745421.0
(22) Date of filing: 28.05.2004
(51) Int. Cl.: A61K 31/045, A61K 31/015, A61K 31/05, A61K 31/122, A61K 31/215, A61K 31/341, A61K 31/428, A61K 31/365, A61K 8/18, A61K 8/99, A61P 25/18, A61P 43/00, A23L 1/30, C11B 9/00

(54) **ANTISTRESS AGENT**

(30) Priority: 30.05.2003 JP 2003155597; 29.08.2003 JP 2003308041
(71) Applicant: SUNTORY LIMITED, Osaka-shi, Osaka 530-8203 (JP)
(72) Inventor: AOSHIMA, Hitoshi, Yamaguchi-shi, Yamaguchi 7530214 (JP); KODA, Hirofumi, Ibaraki-shi, Osaka 5670009 (JP); SHEIKH, Julfikar, Hossain, Khulna (BD)
(74) Representative: Vossius & Partner
(86) International application number: PCT/JP2004/007385
(87) International publication number: WO 2005/011718

(57) **Abstract**

The present invention provides an anti-stress agent which can effectively prevent or alleviate various symptoms caused by stress and which is safe and easily taken or ingested. The anti-stress agent comprises an aroma component of coffee or teas as an active ingredient.

## Description

### TECHNICAL FIELD

The present invention relates to an anti-stress agent using an aroma component contained in coffee or teas, for example, oolong tea. Also, the present invention relates to a medicament or a functional food which contains an aroma component contained in coffee or teas, for example, oolong tea, and which prevents or alleviates mental and physical diseases caused by stress. In addition, the present invention relates to a cosmetic or an aromatic using an aroma component contained in coffee or teas, for example, oolong tea.

### BACKGROUND ART

In the advanced and complex modern society operating around-the-clock, people are exposed to a variety types of physicochemical, psychological and social stress. In particular, for modern people living in complicated human relationships, psychological factors constitute a major part of stress.

Various studies have been conducted concerning psychological stress and various symptoms caused by it. For example, it is reported that, when psychological stress is sensed by cerebrum, noradrenaline release is accelerated in many parts of the brain, and this leads to emotional manifestations such as anxiety and tension (Non-Patent Literature 1). When stress lasts for a long period, various organs in the body are affected and, as a result, severe cases of psychosomatic disorder, depression, gastric ulcer, hypertension, etc. may be caused.

Drugs which are currently used for alleviation of stress or for prevention or treatment of a disease caused by stress are mainly benzodiazepine antianxiety agents and hypnotics. The central GABA nervous system is thought to be a site on which benzodiazepine antianxiety agents and hypnotics act. GABA_{A} receptor, presenting in the GABA nervous system, forms a complex with Cl channel as a center, also with benzodiazepine receptor and barbital-binding site. It is reported that, when benzodiazepine receptor and barbital-binding site are stimulated, affinity of GABA_{A} receptor for GABA is increased (Non-Patent Literature 2).

It is also reported that, when the central GABA nervous system is activated, stress relaxing action is manifested. When a rat is exposed to cold-restraint stress (4°C, 2 hours), gastric ulcer develops. It is reported that this development of gastric ulcer is suppressed in a dose dependent manner by intraventricular administration of GABA (5, 10, 20, 50 µg) and Muscimol (5, 10 µg) which is an agonist of GABA_{A} receptor, and that bicuculine (40 µg) which is an antagonist of GABA receptor diminishes the inhibitory effect of GABA on stress ulceration (Non-Patent Literature 3). Furthermore, it is reported that hypermotionality and gastric ulceration due to stress in rats are inhibited by aminooxy-acetic acid (GABA deaminase inhibitor) which increases the concentration of muscimol, an agonist of GABA_{A} receptor, or central GABA (Non-Patent Literature 4). As described above, the central GABA nervous system plays an important role in relaxing stress.

Although, benzodiazepine antianxiety agents and hypnotics are drugs having relatively high safety, it is not preferable to take them frequently for transient stress in daily lives since they have both habituation and side effects. In addition, when the use of them is discontinued after dosing, a rebound occurs, and thus the symptom could be rather exacerbated in some cases. Therefore, it is difficult to say that they are ideal anti-stress agents.

In view of increasing stress load in modern society and serious influences of stress not only on mental health but on a living body, the development of an anti-stress agent which is truly effective and safe has been desired. In particular, from a prophylactic point of view, the development of an anti-stress agent which can be utilized in foods and luxury goods is desired.

Meanwhile, it is reported that aroma of luxury goods such as coffee increases α wave frequency and gives a relaxing feeling in human beings (Non-Patent Literature 5). However, an active ingredient thereof has not been specified.
(Non-Patent Literature 1) Masatoshi Tanaka, Metabolism, vol.26, p122-131, 1989
(Non-Patent Literature 2) D.G Nicholls, Amino Acids as Neurotransmitter, in "Proteins, Transmitters and Synapses". Blackwell Scientific Publication, Oxford, p.155-185, 1994
(Non-Patent Literature 3) KP Bhargava, GP Gupta and MB Gupta, "Central GABA-eragic mechanism in stress-induced gastric ulceration", British Journal of Pharmacology, vol.84, p.619-623, 1985
(Non-Patent Literature 4) Psychopharmacology 89:47 2-476, 1986
(Non-Patent Literature 5) Life and Health, August, 2000

### DISCLOSURE OF THE INVENTION

An object of the present invention is to provide an anti-stress agent which can effectively prevent or alleviate various symptoms caused by stress, and which is safe and easily taken or ingested. Also, the aroma component contained in coffee or teas, for example, oolong tea according to the present invention is useful as a cosmetic or an aromatic since it is volatile and has an anti-stress activity.

The present inventors investigated various compounds by trial and error in order to develop an effective and safe anti-stress agent and, as a result, they have found unexpectedly that an aroma component of coffee and teas exhibits anti-stress activity.

More particularly, the present inventors conducted screening of substances having anti-stress activity using, as an index, the enhancing effect on GABA response of an oocyte of Xenopus in which GABA receptor is expressed. As a result, the present inventors have found that an aroma component of coffee and teas has activity to enhance GABA response of GABA receptor. Further, the present inventors conducted in vivo pharmacological study using, as an index, the activity to extend duration of the sleeping time when pentobarbital which is GABA receptor-activating drug is administered to mice, and they have found that pentobarbital which is an aroma component of coffee or teas remarkably extends the duration of the sleeping time. As described above, since the central GABA nervous system plays an important role in relaxing stress, aroma components of coffee or teas have an effect to alleviate stress via the activation of central GABA receptor.

Moreover, since people have habitually drunk coffee and teas for a long time, an anti-stress agent containing an aroma component of coffee or teas has no safety problem.

That is, the present invention relates to:
(1) an anti-stress agent comprising an aroma component of coffee or teas as an active ingredient,
(2) the anti-stress agent according to the above (1), wherein the aroma component is one or more ingredients selected from the group consisting of 2-methyl-3-buten-2-ol, 3-methyl-2-buten-1-ol, 1-penten-3-ol, (E)-2-hexen-1-ol, 1-octen-3-ol, sotolone, 2,4-dimethylstyrene, benzothiazole, 2,3,5-trimethylphenol, cis-jasmone, methyl jasmonate, jasmine lactone and linalool oxide,
(3) the anti-stress agent according to the above (1), wherein the tea is oolong tea,
(4) the anti-stress agent according to the above (1), wherein the aroma component is one or more component (s) selected from the group consisting of 1-octen-3-ol, cis-jasmone and methyl jasmonate,
(5) the anti-stress agent according to any one of the above (1) to (4), wherein the amount of the aroma component per dose or per intake is 5 to 50 mg,
(6) the anti-stress agent according to any one of the above (1) to (5), which is a medicament,
(7) the anti-stress agent according to any one of the above (1) to (5), which is a functional food,
(8) the anti-stress agent according to any one of the above (1) to (5), which is a cosmetic,
(9) the anti-stress agent according to any one of the above (1) to (5), which is an aromatic,
(10) the anti-stress agent according to any one of the above (1) to (9), which is a GABA receptor-activating agent,
(11) a method of preventing or alleviating stress, comprising administering an aroma component contained in coffee or teas to a mammal, and
(12) use of an aroma component contained in coffee or teas for the production of a medicament which prevents or alleviates stress.

The anti-stress agent of the present invention can effectively prevent or alleviate stress via activation of GABA receptor, particularly, activation of the central GABA nervous system. Accordingly, the anti-stress agent of the present invention can suppress or prevent various symptoms caused by stress.

Also, since the anti-stress agent of the present invention contains an aroma component of coffee and teas such as oolong tea as an active ingredient, the agent has advantages that it is excellent in safety and has no special limitation upon in use.

Further, since the anti-stress agent of the present invention contains an aroma component of luxury goods such as coffee and teas, the agent is easily taken or ingested. In particular, when the anti-stress agent of the present invention is formulated into a functional food such as a health drink, the agent can be used on a daily basis in order to prevent and/or alleviate stress. In addition, an aroma component of coffee or teas, for example, oolong tea may be used as a cosmetic or an aromatic since it is volatile, and has not only a comfortable fragrance but also an anti-stress activity.

### BRIEF DESCRIPTION OF THE DRAWINGS

Fig. 1 is a graph showing in vitro GABA receptor-activating activity of various aroma components contained in coffee or teas.
Fig. 2 is a graph showing the dose dependency of GABA receptor-activating activity of 1-octen-3-ol.
Fig. 3 is a graph showing the influence on duration of the sleeping time due to pentobarbital when 1-octen-3-ol is orally administered to mice. 1-OCOL (20) shows a group in which 20 mg/kg of 1-octen-3-ol was administered, 1-OCOL (100) shows a group to which 100 mg/kg of 1-octen-3-ol was administered, and CONT shows a control group.
Fig. 4 is a graph showing in vitro GABA receptor-activating activity of various aroma components contained in oolong tea. In the figure, cis-jasmone, methyl jasmonate, jasmine lactone and linalool oxide are represented by cis-JSM, Me-JSM, JSM-LAC and LINL-OX, respectively.
Fig. 5 is a graph showing the dose dependency of GABA receptor-activating activity of cis-jasmone and methyl jasmonate.
Fig. 6 is a graph showing the influence on the duration of the sleeping time due to pentobarbital when mice aspirate cis-jasmone and methyl-jasmonate. * indicates p<0.05 relative to a control.

### BEST MODE FOR CARRYING OUT THE INVENTION

The present invention provides an anti-stress agent containing an aroma component of coffee and teas. Examples of the teas which can be used in the present invention include an unfermented tea, for example, a steamed tea such as Sencha (most popular green tea in Japan), Hojicha (roasted tea), *Gyokuro* (refined green tea grown in the shade), kabusecha (green tea grown in the shade for shorter period than *Gyokuro*) and *Tencha* (green tea made in the same way as *Gyokuro* except that it was dried without rolling), or a pan-fired tea such as *Ureshinocha* (green tea produced in Saga, Japan), *Aoyagicha* (blue-tinted tea produced in Miyazaki, Japan) or various Chinese teas; a semi-fermented tea such as *Bao Zhon* tea (produced in wen shan region, Taiwan), Tie Kuan Yin tea and oolong tea; a fermented tea such as black tea, *Awabancha* (coarse tea produced in Tokushima, Japan), *Goishicha* (coarse tea produced in Tosa, Japan) and Puer tea; and *Genmaicha* (coarse tea mixed with roasted and popped rice), herb tea, turmeric tea, roasted barley tea, roasted adlay tea, mate tea, etc. Among the aforementioned teas, oolong tea is preferable because it contains a large amount of an effective aroma component used in the present invention.

The aroma components contained in the anti-stress agent of the present invention may be those purified from coffee and teas using a known method such as column chromatography, or may be an unpurified concentrate. As the said aroma component, a commercially available purified product may be used. Further, a chemically synthesized aroma component may be used. A plurality of aroma components contained in coffee and teas may be included in the mixed state or only a single aroma component may be included in the anti-stress agent of the present invention.

The aroma component in coffee and teas is not particularly limited, and may be a known ingredient. Specifically, preferable examples of the aroma component include 2-methyl-3-buten-2-ol, 3-methyl-2-buten-1-ol, 1-penten-3-ol, (E)-2-hexen-1-ol, 1-octen-3-ol, sotolone, 2,4-dimethylstyrene, benzothiazole, 2,3,5-trimethylphenol, cis-jasmone, methyl jasmonate, jasmine lactone, linalool oxide and the like. These ingredients are known to be contained in coffee and black tea as an aroma component (Food Rev. Int. , 1989, 5, 317-414). Among those aroma components, 1-octen-3-ol, cis-jasmone and methyl jasmonate are preferable. Those aroma components may be commercially available products, synthetic products or extraction products, and the synthetic method and extraction method may be a known method.

Among teas, it is known that oolong tea contains a large amount of the aforementioned aroma components. For example, jasmine lactone is contained in Long Jing tea (Chinese green tea) only at 0.33 ppm while it is contained in Huang jing gui which is a kind of oolong tea at 26.43 ppm. Also, methyl jasmonate is not detected in Long Jing tea which is one of green teas, but is contained in Hoang jing gui which is one of oolong teas at 3. 33 ppm (Keiichiro Muramatsu, Function of Tea, Gakkaishuppan Center, 2002). It is reported that jasmine lactone is generated during a fermentation process of oolong tea, and jasmine lactone is 50-fold increased by fermentation of oolong tea (J. Agric. Food Chem. 2001, 49, 5391-5396). However, in the case of black teas in which fermentation is further progressed, Darjeeling tea contains only 0.74 ppm jasmine lactone. In addition, methyl epijasmonate which is an analogue of methyl jasmonate is contained in black teas at around 1 ppm at the maximum (Michiko kawakami, Study Note of Tea Perfume, Koseikan, 2000). It should be construed that, in the present invention, methyl jasmonate includes methyl epijasmonate.

When the aroma component of coffee and teas contained in the anti-stress agent of the present invention is a free acid or base, the said aroma component may be contained in a form of a pharmaceutically acceptable salt. Examples of the pharmaceutically acceptable salt include salts with pharmaceutically acceptable acids (e.g. inorganic acid or organic acid) or salts with pharmaceutically acceptable salts with pharmaceutically acceptable bases (e.g. inorganic base or organic base). More specific examples include salts with inorganic acids such as hydrochloric acid, sulfuric acid, phosphoric acid and hydrobromic acid; organic acids such as oxalic acid, maleic acid, fumaric acid, malic acid, tartaric acid, citric acid and benzoic acid; inorganic bases such as alkali metals (e.g. sodium, potassium) and alkaline earth metals (e.g. magnesium, potassium); and organic bases such as organic amines (e.g. triethylamine) basic amino acids (e.g. arginine).

The anti-stress agent of the present invention refers to a substance having anti-stress activity which relaxes or calms stress and maintains homeostasis of a living body, or a composition containing the said substance. In other words, the anti-stress agent of the present invention refers to a substance causing biological reactions to defend against stress or to adapt to stress, or a composition containing the said substance. Here, stress usually refers to strain generated in mind or body when the stimulation causing stress (stressor) is imposed to a living body. Examples of the stressor include physical stimulation such as coldness, change in the weather, radiation and noise; chemical stimulation such as drug, vitamin deficiency and oxygen deficiency; biological stimulation such as bacterial infection; physical stimulation derived from disease or disorder such as pain and fever; mental stimulation arising from human relationship in society and at home such as tension, anxiety and fear.

Therefore, the anti-stress agent of the present invention is effective for preventing stress or promoting recovery from stress, and thus can prevent, improve or cure medical symptoms caused by stress.

Examples of the medical symptoms caused by stress include (a) circulatory diseases such as arteriosclerosis, ischemic heart disease (angina, cardiac infarction), essential hypertension, cardiac neurosis and arrhythmia, (b) respiratory diseases such as bronchial asthma, hyperpnea syndrome and nervous cough, (c) digestive diseases such as digestive ulcer, ulcerative colitis, hypersensitive bowel syndrome, nervous anorexia, nervous vomiting, abdominal bloating and aerophagia, (d) endocrine metabolism diseases such as obesity, diabetes, psychogenic polyposia and Basedow's disease, (e) nervous diseases such as migraine, muscle contraction headache and autonomic imbalance (f) urologic diseases such as nocturnal enuresis, impotence and neurogenic bladder, and (g) bone and muscle diseases such as rheumatoid arthritis, systemic myalgia and spinal irritation.

Furthermore, examples of the medical symptoms caused by the stress include (h) skin diseases such as nervous dermatitis, alopecia areata, hyperhidrosis and eczema, (i) otorhinolaryngological diseases such as Ménière syndrome, foreign body sensation in the throat, hearing disorder, susurrus aurium, motion sickness and aphasia dysphemia, (j) ophtalmologic diseases such as primary glaucoma, asthenopia, blepharospasm and ocular hysteria, (k) obstetric and gynecological diseases such as dysmenorrhea, amenorrhea, menstrual disorder, dysfunctional uterine bleeding, menopausal disorder, frigidity and infertility, (1) pediatric diseases such as orthostatic disturbance, recurrent umbilical colic, psychogenic fever and pavor nocturnus, (m) conditions before and after operation such as intestinal adhesion, dumping syndrome, polysurgery and neurosis after plastic surgery, (n) oral cavity diseases such as idiopathic glossalgia, some types of stomatitis, ozostomia, abnormal salivation, masseter check and denture neurosis, (o) neurosis and (p) depression.

The anti-stress agent of the present invention can be used as a medicament or a functional food. When the anti-stress agent of the present invention is used as a medicament, such medicament may be in any dosage form as long as oral administration or parenteral administration is advantageously conducted. Examples of a dosage form of the medicament of the present invention include injectables, infusions, powders, granules, tablets, capsules, enteric coated agents, troaches, solutions for internal use, suspensions, emulsions, syrups, solutions for external use, fomentations, nasal drops , ear drops, eye drops, inhalants, ointment, lotions and suppositories. Also, in the present invention, the medicaments of the present invention in the aforementioned dosage forms may be used solely or in combination of them depending on the symptom.

In the medicament of the present invention, the known additives which can be usually used in the pharmaceutical art such as excipients, binders, disintegrating agents, lubricants, corrigents and stabilizers may be added to the principal agent depending on the purpose.

More specifically, when the medicament of the present invention is solid preparations such as capsules, tablets, powders and granules, additives, for example, excipients such as lactose, glucose, sucrose and mannitol; disintegrating agents such as starch and sodium alginate; lubricants such as magnesium stearate and talc; binders such as polyvinyl alcohol, hydroxypropylcellulose and gelatin; surfactants such as fatty acid ester; plasticizers such as glycerin may be contained in the preparations. On the other hand, when the medicament of the present invention is liquid preparations, additives, for example, sugars such as sucrose, sorbitol, fructose; glycols such as polyethylene glycol and propylene glycol; oils such as sesame oil, olive oil and soybean oil; antiseptics such as p-hydroxybenzoate esters may be contained in the preparations. In addition, in the case of liquid preparations, the medicament of the present invention may be a preparation in such a form that an aroma component which is an active ingredient is dissolved upon use.

The aforementioned medicament of the present invention can be easily prepared by the methods well-known per se or conventional in the pharmaceutical art.

An administration route of the medicament of the present invention is not particularly limited, and may be either oral administration or parenteral administration. Inter alia, from a viewpoint of easy ingestion, a medicament of the present invention which can be administered orally is preferable.

A dose of the medicament of the present invention varies depending on administration route, dosage form, and severity of symptom, age or weight of the patient, thus cannot be generalized. Specifically, the amount per dose for an adult is such that a total amount of the aroma components of the present invention contained in one solid preparation such as capsule is around 5 to 500 mg, preferably around 5 to 50 mg.

When the anti-stress agent of the present invention is used as a functional food, a form thereof may be the same as that of the aforementioned pharmaceutical preparations. Alternatively, the food may be processed into a form such as natural liquid foods, semi-digested nourishing foods, component nourishing foods and drinks. Further, the functional food of the present invention may be formulated into easily soluble preparations which are added to alcohol drinks or mineral water at the time of use. More specifically, examples of the form of the functional food of the present invention include a confectionery such as biscuit, cookie, cake, candy, chocolate, chewing gum and Japanese sweets; bread, noodles, rice and bean curd, or a processed product thereof; fermented foods such as refined rice wine and alcoholic beverage with medical properties; seasonings such as *Mirin* (Japanese sweet rice wine for cooking), edible vinegar, soy sauce, *Miso* (fermented bean paste) and dressing; livestock food products such as yogurt, ham, bacon, sausage and mayonnaise; processed marine products such as boiled fish paste, fried fish paste and *Hampen* (fish paste cake); drinks such as fruit drinks, soft drinks, isotonic drinks, alcohol drinks, tea, coffee and cocoa. When the aroma component of the present invention is used by adding to drinks, the content is preferably 0.0005 to 1.0 wt% per 1 L of the drink and the amount per intake is around 5 to 500 mg, preferably around 5 to 50 mg.

Alternatively, the functional food of the present invention may be provided to a patient in a form of a functional food prepared in situ by adding the anti-stress agent of the present invention to an arbitrary food in the preparation of hospital meals under the supervision of a dietitian based on the food prescription of a doctor. The anti-stress agent of the present invention may be liquid, or solid such as powder and granule.

The functional food of the present invention may contain supplemental ingredients which are common in the food field. Examples of the supplemental ingredient include lactose, sucrose, liquid sugar, honey, magnesium stearate, oxypropylcellulose, various vitamins, microelements, citric acid, malic acid, perfume and inorganic salt.

The functional food of the present invention may be taken when stress is expected to occur, or when experiencing stress, for example, during physical labor, mental work and sports. Alternatively, the functional foods may be used regularly in order to prevent or alleviate stress.

The amount of intake of the functional food of the present invention varies depending on the status of stress, age, sex, etc. of a person who ingests the food, thus cannot be generalized. Specifically, the amount per intake for an adult is such that a total amount of the aroma components of the present invention is around 5 to 500 mg, preferably around 5 to 50 mg.

The medicament or the functional food of the present invention may be used in combination with other anti-stress agent, nutritional agent such as vitamin, hormone preparation, etc., microelement or iron compound. For example, when the functional food of the present invention is a health drink, if desired, the nature of luxury drink can be preferably imparted to the drink by mixing with other physiologically active ingredient, mineral, hormone, nutritional ingredient, flavor or the like.

The effect that stress has on human beings includes depressive tendencies. This tendency is often recognized in normal daily life before it gets pathological state. Accordingly, providing a means to alleviate the tendency has been an important challenge to be addressed. Recently, aromatherapy, in particular, has attracted attention as a means to alleviate depressive tendencies. The aroma component of the present invention, which is volatile and has a comfortable aroma, may be used as a cosmetic or an aromatic.

The aroma component of coffee or teas according to the present invention may be used as a cosmetic or an aromatic including, for example, body care products such as perfume, *eau* de toilette, cologne, antiperspirant, deodorant, shampoo, hair rinse, hair conditioner, hair treatment, hair grooming agent, soap, body soap, basic skin care product, etc; laundry products such as laundry detergent, household cleaner, household fabric softener, laundry bleach, etc.; household products such as dish detergent, toilet cleaner, household bleach, aromatic, floor polish, etc.; oral care products such as toothpaste, mouthwash, breath cleaner, breath refresher, etc. ; and sundry products such as cataplasm, etc.

Optional cosmetic components used in the cosmetics field such as oil and fat, moisturizing agent, natural dye, surfactant, antioxidant, antiseptic, etc. may be used for the cosmetic containing the aroma component of coffee and teas according to the present invention, and said cosmetic may be produced by a method known *per se.* Use of the cosmetic of the present invention will result in effects of freeing people from the physiological mental conditions which they experience in daily life such as drowsiness, fatigue, lassitude, etc., refreshing feeling, and revitalizing mental activities. The amount of the aroma component contained in a cosmetic is generally 0.005 to 10% by weight of the cosmetic as a whole although it is decided depending on the form and the purpose of use of the cosmetic.

Furthermore, the aromatic of the present invention may be contained in containers known per se for aromatics and may be used. The form of the aromatic includes spray and solid (granule), and such aromatics can create a pleasant-smelling space. The amount of the aroma component used in the aromatic is generally 0.005 to 10% by weight of the aromatic as a whole, although it is decided depending on the place to be used or the necessary strength of the aroma.

### EXAMPLES

The following Examples further illustrate the present invention, but the scope of the present invention is not limited by them.

### Example 1

### In vitro GABA receptor-activating activity of aroma components (9 kinds)

As an experimental material, an oocyte of Xenopus was used. A rat-derived GABA receptor mRNA was injected into the nucleus of an oocyte to express GABA receptor on the surface of the egg. The oocyte was cultured in a normal Ringer's solution for frog (115 mM NaCl, 1 mM KC1, 1.8 mM CaCl₂, 5 mM Tris, pH 7.2). Using the resulting oocyte, inhibitory potential of GABA (10 µM) and inhibitory potential of a mixed solution of GABA (10 µM) and the below mentioned sample were measured. Generation of inhibitory potential was measured by voltage clamp method using a voltage clamping amplifier (CEZ-1100; manufactured by NIHON KODEN CORPORATION).

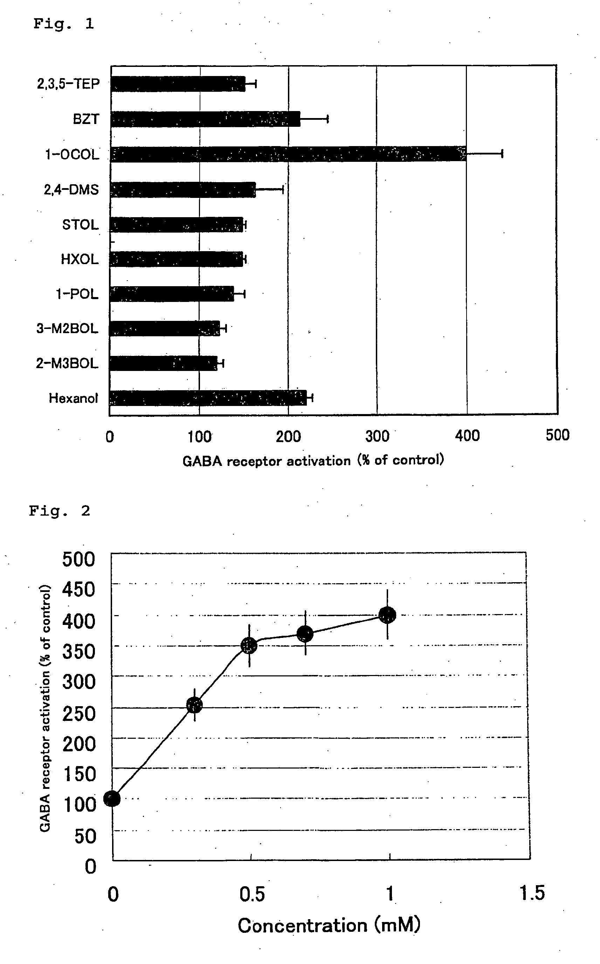

As a sample, the following 9 kinds of aroma components contained in coffee or teas were used in a concentration of 2 µl/ml. Samples used are as follows: 2-methyl-3-buten-2-ol (expressed by 2-M3BOL in Fig. 1), 3-methyl-2-buten-1-ol (expressed by 3-M2BOL in Fig. 1), 1-penten-3-ol (expressed by 1-POL in Fig. 1), (E)-2-hexen-1-ol (expressed by HXOL in Fig. 1), 1-octen-3-ol (expressed by 1-OCOL in Fig. 1), sotolone (expressed by STOL in Fig. 1), 2,4-dimethylstyrene (expressed by 2,4-DMS in Fig. 1), benzothiazole (expressed by BZT in Fig. 1) and 2,3,5-trimethylphenol (expressed by 2,3,5-TEP in Fig. 1). The aforementioned samples used in the experiment were commercially available products. That is, sotolone manufactured by Ogawa & Co., Ltd. and other compounds manufactured by Tokyo Kasei Co., Ltd. were used.

The results are shown in Fig. 1. As an index, enhancing activity of the sample on inhibitory potential induced by GABA was used. The results are expressed by % increase of the inhibitory potential when GABA and a sample were used in combination, relative to the inhibitory potential generated when only GABA (0.25 µM) was added (set at 100%). As apparent from Fig. 1, it was found that all of assessed samples enhanced GABA response to GABA receptor, and 1-octen-3-ol in particular had a strong activity to enhance the response.

In Example 1, hexanol was used as a positive control. It is known that hexanol as a component of phytoncide has GABA receptor-activating activity (Biosci. Biotechnol. Biochem., 63, 743-748, 1999) and stress relaxing activity (1998-1999 Scientific Research Subsidy, Fundamental Research (C) (2) Research Outcome Report, Research Theme No.10670070).

### Example 2

### Study on dose dependency of 1-octen-3-ol

Dose dependency of 1-octen-3-ol which had been recognized to have a strong activity in Example 1 was studied. The experimental procedures were entirely the same as those of Example 1. The concentration of GABA was 5 µM, and the concentration of 1-octen-3-ol was 0.3 to 1 mM. The results were shown in Fig. 2. As apparent from Fig. 2, 1-octen-3-ol showed up to 400 % of GABA response to GABA receptor. The results shown in Fig. 2 are expressed by the relative value of the inhibitory potential when GABA and 1-octen-3-ol were used in combination, compared to the inhibitory potential generated when only GABA (5 µM) was added (set at 100%).

### Example 3

### Enhancing Activity of 1-octen-3-ol on pentobarbital-induced sleep

As a test animal, ddy male mice (8-week age) were purchased from Shimizu Laboratory Supplies Co., Ltd., pre-bred for one week and used in the experiment. Each five of the mice were bred in a polyisopentene cage (manufactured by Clea Japan. Inc.) in a breeding chamber where the breeding conditions were set at room temperature of 23 ±2°C, humidity of 55 ±5%, ventilation frequency of 12 to 15/hour (all fresh air ventilation), and lighting hours of 12 hours/day (from 7 a.m. to 7 p.m.). The mice were fed solid feed CE-2 (Clea Japan Inc.) and were allowed free access to water.

1-Octen-3-ol dissolved in olive oil was orally administered to a mouse at a dose of 20 or 100 mg/kg. In each group, 8 mice were used. Thirty minutes later, pentobarbital dissolved in physiological saline was intraperitoneally administered at a dose of 50 mg/kg, and duration of the sleeping time was measured. The sleeping time was defined as the time period from the loss of righting reflex until its recovery.

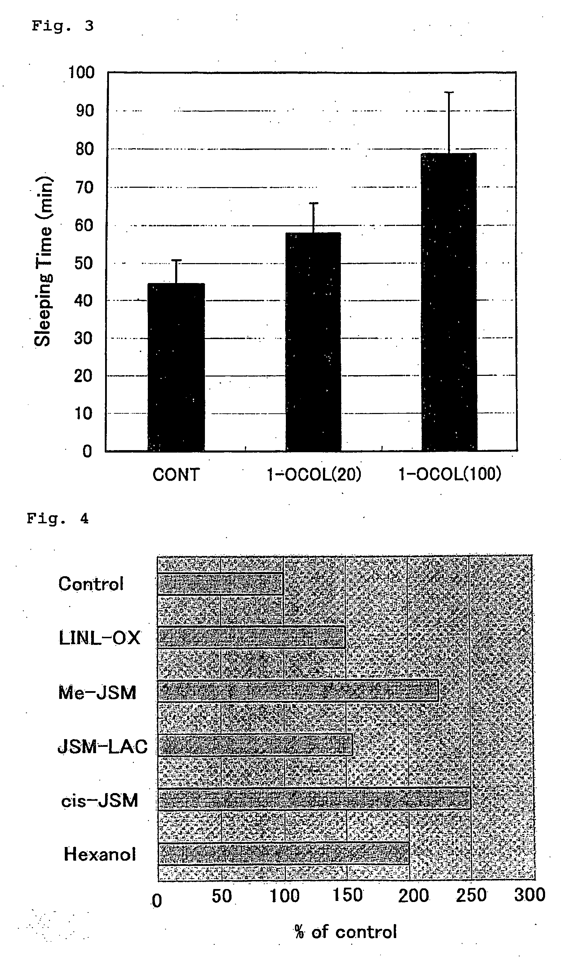

The results are shown in Fig. 3. The experimental results shown in Fig. 3 are expressed by average ± standard error, and Student t-test was used in a significant difference test. As apparent from Fig. 3, 1-octen-3-ol extended duration of the sleeping time induced by pentobarbital in a dose dependent manner.

### Example 4

### In vitro GABA receptor-activating activity of aroma component of luxury goods (oolong tea)

As an experimental material, an oocyte of Xenopus was used. A rat-derived GABA receptor mRNA was injected into the nucleus of an oocyte to express GABA receptor on the surface of an egg. The oocyte was cultured in a normal Ringer's solution for frog (115 mM NaCl, 1 mM KCl, 1.8 mM CaCl₂, 5 mM Tris, pH 7.2). Generation of the inhibitory potential induced by GABA (0.25 µM) was measured by voltage clamp method using a voltage clamping amplifier (CEZ-1100; manufactured by Nihon Koden corporation).

As samples, the below-mentioned 4 kinds of aroma components of oolong tea were used in a concentration of 0.5 mM. The samples used are as follows: cis-jasmone, methyl jasmonate, jasmine lactone and linalool oxide.

The said samples used in the experiment were those manufactured by Ogawa & Co., Ltd.

The results are shown in Fig. 4. As an index, enhancing activity of the sample on inhibitory potential induced by GABA was used. The results are expressed by % increase of the inhibitory potential when GABA and a sample were used in combination, relative to the inhibitory potential generated when only GABA (0.25 µM) was added (set at 100%). As apparent from Fig. 4, it was found that all of assessed samples enhanced GABA response to GABA receptor and cis-jasmone and methyl jasmonate in particular had an activity to enhance the response.

In Example 4, hexanol was used as a positive control. It is known that hexanol as a component of phytoncide has GABA receptor-activating activity (Biosci. Biotechnol. Biochem., 63, 743-748, 1999) and stress relaxing activity (1998-1999 Scientific Research Subsidy, Fundamental Research (C) (2) Research Outcome Report, Research Theme No.10670070).

### Example 5

### Study on dose dependency of cis-jasmone and methyl jasmonate

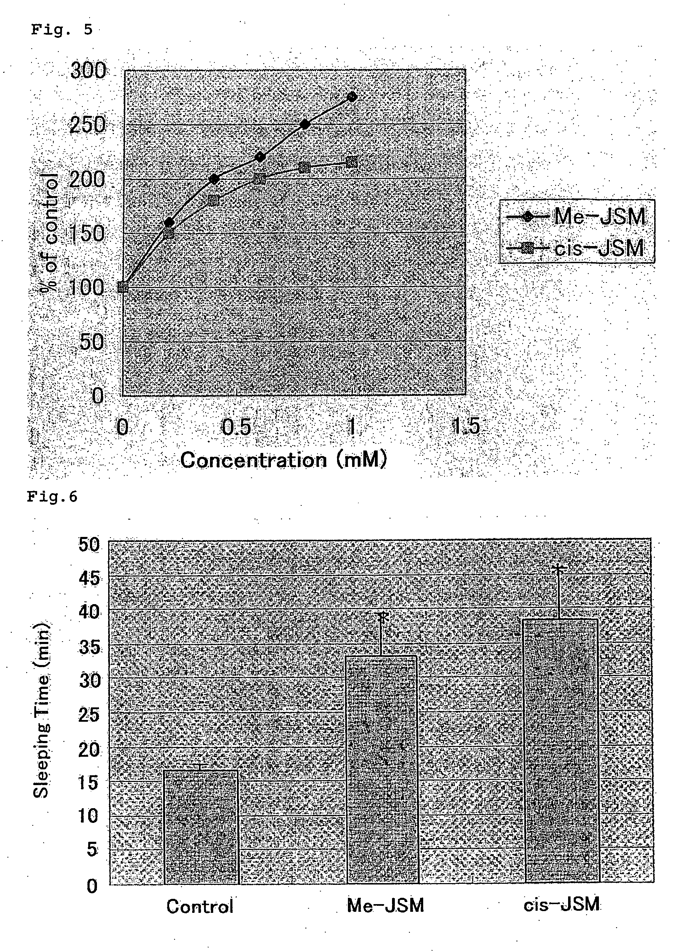

Dose dependency of cis-jasmone and methyl jasmonate which had been recognized to have a strong activity in Example 4 was studied. The experimental procedures were entirely the same as those of Example 4. The concentration of GABA was 5 µM, and the concentrations of cis-jasmone and methyl jasmonate were 0.2 to 1 mM. The results are shown in Fig. 5. As apparent from Fig. 5, cis-jasmone and methyl jasmonate showed up to 200 % and 300 %, respectively of GABA response to GABA receptor. The results shown in Fig. 5 are expressed by the relative value of the inhibitory potential when GABA and cis-jasmone and methyl jasmonate were used in combination compared to the inhibitory potential generated when only GABA (5 µM) was added (set at 100%).

### Example 6

### Enhancing activity of cis-jasmone and methyl jasmonate on pentobarbital-induced sleep

As a test animal, ddy male mice (8-week age) were purchased from Shimizu Laboratory Supplies Co., pre-bred for one week, and used in the experiment. Each five of the mice were bred in a polyisopentene cage (manufactured by Clea Japan. Inc.) in a breeding chamber where the breeding conditions were set at room temperature of 23 ±2°C, humidity of 55 ±5%, ventilation frequency of 12 to 15/hour (all fresh air ventilation) and lighting hours of 12 hours a day (from 7 a.m. to 7 p.m.). The mice were fed solid feed CE-2 (Clea Japan. Inc.) and were allowed free access to water.

A mouse was made to aspirate cis-jasmone and methyl jasmonate diluted to 0.1% in an exposure box until the completion of the experiment. In each group, 5 mice were used. Thirty minutes after initiation of aspiration, pentobarbital dis solved in physiological saline was intraperitoneally administered at a dose of 50 mg/kg, and duration of the sleeping time was measured. The sleeping time was defined as the time period from the loss of righting reflex until its recovery.

The results are shown in Fig. 6. The experimental results shown in Fig. 6 are expressed by average ± standard error, and Student T-test was used in a significant difference test. As apparent from Fig. 6, cis-jasmone and methyl jasmonate significantly extended duration of the sleeping time induced by pentobarbital.

### Preparation Examples

### Preparation Example 1

### Capsule

### a. Formulation (per 500 mg)

1-Octen-3-ol: 5 mg
Lactose: 493 mg
Magnesium stearate: 2 mg

### b. Process

According to the above formulation, 1-octen-3-ol and lactose were mixed, compressed and ground. Into this was mixed magnesium stearate at the formulation amount. Each 500 mg of the mixture was filled into a No. 2 capsule to prepare a capsule containing 5 mg of 1-octen-3-ol per capsule.

### Preparation Example 2

### Health drink

### a. Formulation

1-Octen-3-ol: 5 g
Sodium DL-tartarate: 0.1 g
Succinic acid: 9 mg
Liquid sugar: 800 g
Citric acid: 12 g
Vitamin C: 10 g
Perfume: 12 ml
Potassium chloride: 1 g
Magnesium sulfate: 0.5 g

### b. Process

According to the formulation, the above ingredients were dissolved in 8 L of distilled water, distilled water was added thereto to give a total amount of 10L. The resulting solution was passed though a 0.22 µm sterilization filter, and each 100 ml of which was sterilely filled into a brown bottle to prepare a health drink containing 5 mg of 1-octen-3-ol per agent.

### Preparation Example 3

### Capsule

### a. Formulation (per capsule)

| | |
|---|---|
| Methyl jasmonate: | 5 mg |
| Lactose: | 493 mg |
| Magnesium stearate: | 2 mg |
| | 500 mg |

### b. Process

According to the formulation, methyl jasmonate and lactose weremixed, compressed and ground. Into this was mixed magnesium stearate at the formulation amount. Each 500 mg of the mixture was filled into a No. 2 capsule to prepare a capsule containing 5 mg of methyl jasmonate per capsule.

### Preparation Example 4

### Preparation of a health drink

### a. Formulation

cis-jasmone: 5 g
Sodium DL-tartarate: 0.1 g
Succinic acid: 9 mg
Liquid sugar: 800 g
Citric acid: 12 g
Vitamin C: 10 g
Perfume: 12 ml
Potassium chloride: 1 g
Magnesium sulfate: 0.5 g

### b. Process

According to the formulation, the above ingredients were dissolved in 8 L of distilled water, and distilled water was added thereto to give a total amount of 10L. The resulting solution was passed through a 0.22 µm sterilization filter, and each 100 ml of which was sterilely filled into a brown bottle to prepare a health drink containing 5 mg of cis-jasmone per agent.

### INDUSTRIAL APPLICABILITY

The anti-stress agent of the present invention can inhibit or prevent various symptoms accompanied with stress, and is useful as a medicament, a functional food, a cosmetic or an aromatic.

## Claims

1. An anti-stress agent comprising an aroma component of coffee or teas as an active ingredient.

2. The anti-stress agent according to claim 1, wherein the aroma component is one or more component(s) selected from the group consisting of 2-methyl-3-buten-2-ol, 3-methyl-2-buten-1-ol, 1-penten-3-ol, (E)-2-hexen-1-ol, 1-octen-3-ol, sotolone, 2,4-dimethylstyrene, benzothiazole, 2,3,5-trimethylphenol, cis-jasmone, methyl jasmonate, jasmine lactone and linalool oxide.

3. The anti-stress agent according to claim 1, wherein the tea is oolong tea.

4. The anti-stress agent according to claim 1, wherein the aroma component is one or more component(s) selected from the group consisting of 1-octen-3-ol, cis-jasmone and methyl jasmonate.

5. The anti-stress agent according to any one of claims 1 to 4, wherein the amount of the aroma component per dose or per intake is 5 to 50 mg.

6. The anti-stress agent according to any one of claims 1 to 5, which is a medicament.

7. The anti-stress agent according to any one of claims 1 to 5, which is a functional food.

8. The anti-stress agent according to any one of claims 1 to 5, which is a cosmetic.

9. The anti-stress agent according to any one of claims 1 to 5, which is an aromatic.

10. The anti-stress agent according to any one of claims 1 to 9, which is a GABA receptor-activating agent.

11. A method of preventing or alleviating stress, comprising administering an aroma component contained in coffee or teas to a mammal.

12. Use of an aroma component contained in coffee or teas for the production of a medicament which prevents or alleviates stress.
